# EUROPEAN PATENT SPECIFICATION

(11) **EP 3 376 227 B1**
(45) Date of publication and mention of the grant of the patent: **21.02.2024**
(21) Application number: 18161647.5
(22) Date of filing: 14.03.2018
(51) Int. Cl.: A61P 35/00, A61P 35/04, A61P 37/06, G01N 33/574, G01N 33/49

(54) **METHODS FOR DIAGNOSING AND TREATING CANCER**
VERFAHREN ZUR DIAGNOSE UND BEHANDLUNG VON KREBS
PROCÉDÉS DE DIAGNOSTIC ET DE TRAITEMENT DU CANCER

(30) Priority: 14.03.2017 US 201762471083 P; 10.11.2017 US 201762584634 P
(43) Date of publication of application: 19.09.2018
(73) Proprietor: MiCareo Taiwan Co., Ltd., 114 Taipei City (TW)
(72) Inventor: Tseng, Ju-Yu, 114 Taipei City (TW); Chen, Yen-Ru, 114 Taipei City (TW); Lee, Chia-Ying, 114 Taipei City (TW); Wu, Li-Fan, 114 Taipei City (TW); Wang, Shin-Hang, 114 Taipei City (TW); Chen, Jui-Lin, 114 Taipei City (TW); Chen, Chwen-Cheng, 114 Taipei City (TW)
(74) Representative: Straus, Alexander

(56) References cited:
- WO-A2-2010/096734
- SALVATORE GRISANTI ET AL: "Circulating Tumor Cells in Patients with Recurrent or Metastatic Head and Neck Carcinoma: Prognostic and Predictive Significance", PLOS ONE, vol. 9, no. 8, 8 August 2014 (2014-08-08), page e103918, XP055487165, DOI: 10.1371/journal.pone.0103918
- JINMING YU ET AL: "PD-L1 expression in human cancers and its association with clinical outcomes", ONCOTARGETS AND THERAPY, vol. Volume 9, 1 August 2016 (2016-08-01), pages 5023-5039, XP055486852, DOI: 10.2147/OTT.S105862
- CHIARA NICOLAZZO ET AL: "Monitoring PD-L1 positive circulating tumor cells in non-small cell lung cancer patients treated with the PD-1 inhibitor Nivolumab", SCIENTIFIC REPORTS, vol. 6, no. 1, 24 August 2016 (2016-08-24) , XP055486605, DOI: 10.1038/srep31726
- STROME SCOTT E ET AL: "B7-H1 blockade augments adoptive T-cell immunotherapy for squamous cell carcinoma", CANCER RESEARCH, AMERICAN ASSOCIATION FOR CANCER RESEARCH, US, vol. 63, no. 19, 1 October 2003 (2003-10-01), pages 6501-6505, XP002487684, ISSN: 0008-5472
- KYLE P. MCMULLEN ET AL: "Circulating tumor cells in head and neck cancer: A review", WORLD JOURNAL OF OTORHINOLARYNGOLOGY-HEAD AND NECK SURGERY, vol. 2, no. 2, 1 June 2016 (2016-06-01), pages 109-116, XP055486769, ISSN: 2095-8811, DOI: 10.1016/j.wjorl.2016.05.003
- ARUTHA KULASINGHE ET AL: "Circulating tumour cells in metastatic head and neck cancers : Circulating tumour cells in metastatic HNC", INTERNATIONAL JOURNAL OF CANCER, vol. 136, no. 11, 11 August 2014 (2014-08-11), pages 2515-2523, XP055486844, US ISSN: 0020-7136, DOI: 10.1002/ijc.29108
- E. SAÂDA-BOUZID ET AL: "Hyperprogression during anti-PD-1/PD-L1 therapy in patients with recurrent and/or metastatic head and neck squamous cell carcinoma", ANNALS OF ONCOLOGY., vol. 28, no. 7, 1 July 2017 (2017-07-01), pages 1605-1611, XP055486761, NL ISSN: 0923-7534, DOI: 10.1093/annonc/mdx178

## Description

### FIELD OF THE INVENTION

The present invention pertains to methods for diagnosing and treating cancer.

### BACKGROUND OF THE INVENTION

Circulating tumor cells (CTCs) presence in circulation play active roles in mediating metastasis[1]. Enumeration of CTCs was reported as a prognostic predictor for metastatic colorectal cancer (mCRC) patients [2]. In most of the previous studies, the number of CTCs was enumerated from blood drawn by venipuncture of the forearm [3, 4]. It was suggested that the presence of circulating tumor cells expressing PD-L1 in non-small cell lung cancer patients at 6 months after the treatment of Nivolumab (PD-1 inhibitor) corresponds to a therapy escape or poor prognosis [5].

Grisanti et al. (PLOS ONE, 2014) discloses a method for detection of circulating tumor cells (CTCs) in patient with recurrent/metastatic head and neck carcinoma before systemic therapy. However, Grisanti et al. does not disclose the method of the detection of PD-L1⁺-CTC for the prognosis and prediction of head and neck cancer. Yu et al. (Oncotargets and Therapy, 2016) discloses that the prognostic vale of PD-L1 expression in cancer is controversial although the overexpression of PD-L1 in cancers is associated with poor clinical outcomes. Yu et al. also teaches PD-L1 expression in cancer as predictive biomarker evaluated in many trials. Tumor PD-L1 expression is an effective predictor of the outcomes of cancer immunotherapy.

However, none of the prior art discloses the method for identifying a subject at risk of developing a recurrent or metastatic cancer, comprising detecting PD-L1⁺ circulating tumor cells in a mesenteric venous blood sample. Until now, there is no promising method or kit for early detection or diagnosis of a metastatic cancer.

### BRIEF SUMMARY OF THE INVENTION

The present invention is based on the unexpected finding that in patients having a gastrointestinal cancer or head and neck squamous-cell carcinoma, the presence/level of circulating tumor cells expressing PD-L1 (called as "PD-L1⁺ circulating tumor cells") before a therapy for, or during a surgery of curative resection of the gastrointestinal cancer or head and neck squamous-cell carcinoma, correlates with the metastasis of cancers or the prognosis of patients.

Accordingly, in one aspect, the present invention provides a method for identifying a subject at risk of developing a recurrent or metastatic cancer, comprising detecting PD-L1⁺ circulating tumor cells in a mesenteric venous blood sample of the subject, wherein the presence of one or more PD-L1⁺ circulating tumor cells indicates that the subject is at risk of developing a recurrent or metastatic cancer, and wherein the subject has a gastrointestinal cancer or head and neck squamous-cell carcinoma and the blood sample is derived from the subject before a therapy for, or during a surgery of curative resection of the gastrointestinal cancer or head and neck squamous-cell carcinoma.

In one embodiment of the present invention, the cancer is a gastrointestinal cancer, particularly a colorectal cancer. In another embodiment, the cancer is head and neck squamous-cell carcinoma.

In another aspect of the present invention the blood sample is derived from the subject during a surgery of curative resection of the gastrointestinal cancer.

In a further aspect, the present invention provides a method for predicting prognosis of a patient having gastrointestinal cancer or head and neck squamous-cell carcinoma, comprising detecting PD-L1⁺ circulating tumor cells in a mesenteric venous blood sample of the patient, wherein the presence of one or more PD-L1⁺ circulating tumor cells indicates poor prognosis or overall survival of the patient, wherein the blood sample is derived from the patient before a therapy for, or during a surgery of curative resection of the gastrointestinal cancer or head and neck squamous-cell carcinoma.

Said treatment may be an immunotherapy. The subject may be administered with an immunotherapy, in combination with a chemotherapy or a targeted therapy.

According to certain embodiments of the present invention, the cancer is a recurrent or metastatic cancer.

In one embodiment of the present invention, the cancer is a gastrointestinal cancer, particularly a colorectal cancer. In another embodiment, the cancer is head and neck squamous-cell carcinoma.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:
**Fig. 1** illustrates the sample preparation for circulating tumor cell (CTC) enumeration via MiSelect R System.
**Fig. 2** shows typical CTC images.
**Fig. 3** shows the comparison of CTC counts in peripheral blood (PB) and mesenteric venous blood (MVB).
**Fig. 4A** shows the results on CTC counts in PB of colorectal cancer (CRC) patients at various stages. **Fig. 4B** shows the results on CTC counts in MVB of CRC patients at various stages.
**Fig. 5** shows the heterogeneity of PD-L1 expressions on CTC.
**Fig. 6** shows PD-L1⁺ CTC counts in PB and MVB.
**Fig. 7** shows PD-L1⁺ CTC counts in MVB at various stages.
**Fig. 8** shows the frequency of occurrence of PD-L1⁺ CTC at various stages.
**Fig. 9** shows the heterogeneity of PD-L1 expressions on CTC

### DETAILED DESCRIPTION OF THE INVENTION

In one aspect, the present invention provides a method for identifying a subject at risk of developing a recurrent or metastatic cancer, comprising detecting PD-L1⁺ circulating tumor cells in a mesenteric venous blood sample of the subject, wherein the presence of PD-L1⁺ circulating tumor cells indicates that the subject is at risk of developing a recurrent or metastatic cancer, and wherein the subject has a gastrointestinal cancer or head and neck squamous-cell carcinoma and the blood sample is derived from the subject before a therapy for, or during a surgery of curative resection of the gastrointestinal cancer or head and neck squamous-cell carcinoma.

According to the present invention, a higher level of the PD-L1⁺ circulating tumor cells indicates a higher risk of developing a recurrent or metastatic cancer.

In one embodiment of the present invention, the cancer is a gastrointestinal cancer, particularly a colorectal cancer. The blood sample is a mesenteric venous blood sample. The mesenteric venous blood sample may be derived during surgeries of curative resection of the gastrointestinal cancer. The gastrointestinal cancer includes but is not limited an esophageal cancer, a gastric cancer, a gastrointestinal stromal tumor, a pancreatic cancer, a liver cancer, a gallbladder cancer, a colorectal cancer, and an anal cancer.

In a particular example of the present invention, the cancer is a colorectal cancer.

In another embodiment, the cancer is head and neck squamous-cell carcinoma.

In a further aspect, the present invention provides a method for predicting prognosis of a patient having a gastrointestinal cancer or head and neck squamous-cell carcinoma, comprising detecting PD-L1⁺ circulating tumor cells in a mesenteric venous blood sample of the patient, wherein the presence of one or more PD-L1⁺ circulating tumor cells indicates poor prognosis or overall survival of the patient, and wherein the blood sample is derived from the patient before a therapy for, or during a surgery of curative resection of the gastrointestinal cancer or head and neck squamous-cell carcinoma.

According to the present invention, a higher level of the PD-L1⁺ circulating tumor cells indicates a worse prognosis of the patient.

The treatment may include but is not limited to immunotherapy, chemotherapy, radiation therapy, hormone therapy, or a combination thereof.

Said treatment may be an immunotherapy. The subject may be administered with an immunotherapy, in combination with a chemotherapy or a targeted therapy.

According to certain embodiments of the present invention, the cancer is a recurrent or metastatic cancer.

In one embodiment of the present invention, the cancer is a gastrointestinal cancer, particularly a colorectal cancer. In another embodiment, the cancer is head and neck squamous-cell carcinoma.

The blood sample is a mesenteric venous blood sample. The mesenteric venous blood sample may be derived during surgeries of curative resection of the gastrointestinal cancer.

In certain embodiments of the present invention, the cancer is a gastrointestinal cancer, which includes, but is not limited to, an esophageal cancer, a gastric cancer, a gastrointestinal stromal tumor, a pancreatic cancer, a liver cancer, a gallbladder cancer, a colorectal cancer, and an anal cancer. In a particular example of the present invention, the cancer is a colorectal cancer.

In some other embodiments of the present invention, the cancer is head and neck squamous-cell carcinoma.

The present invention is further illustrated by the following examples, which are provided for the purpose of demonstration rather than limitation.

### Examples

### Materials and Methods

### 1. Patient characteristics

A total of 116 patients who underwent curative surgical resection at Taipei Veterans General Hospital between April 2016 and September 2017 were enrolled. A total 26 HNSCC, 9 HCC, 5 UC and 2 RCC patients who underwent receiving immunotherapy at Taipei Veterans General Hospital between October 2016 and September 2017 were also enrolled. The enrollment procedures followed the protocols approved by the Internal Review Board of Taipei Veterans General Hospital. All patients provided written informed consent. Patients who prior to colonoscopy examination or suspected of having colorectal cancer (CRC) with unconfirmed clinical stages were recruited. Six patients who had histological diagnosed tubular adenoma were also enrolled. The average (±SD) age of the assessable patients was 63.6 ± 12.5 years (median, 64) (see **Table 1** below). Primary tumor staging was confirmed by histologic examination of the resected primary tumor. Based on histologic examination, the subjects consisted of 116 CRC patients (24 stage I, 38 stage II, 42 stage III and 12 stage IV, respectively).

**Table 1. Clinicopathological characteristics of the study population**

| | Stage I (n=24) | Stage II (n=38) | Stage III (n=42) | Stage IV (n=12) |
|---|---|---|---|---|
| Age Median (range) | 64 (37-79) | 68 (38-92) | 64 (37-84) | 59 (47-90) |
| Gender | | | | |
| Male | 10 | 28 | 22 | 8 |
| Female | 14 | 10 | 20 | 4 |

| Tumor location | | | | |
|---|---|---|---|---|
| Colon | 20 | 27 | 29 | 9 |
| Rectum | 4 | 11 | 13 | 3 |

| T stage | | | | |
|---|---|---|---|---|
| T1 | 14 | 0 | 3 | 0 |
| T2 | 10 | 0 | 5 | 1 |
| T3 | 0 | 24 | 21 | 4 |
| T4 | 0 | 14 | 13 | 7 |

| N stage | | | | |
|---|---|---|---|---|
| N0 | 24 | 38 | 0 | 2 |
| N1 | 0 | 0 | 31 | 4 |
| N2 | 0 | 0 | 11 | 6 |
| CEA (≥ 5 ng/mL) | 3 | 17 | 17 | 10 |
| CA 19-9 (≥ 37 ng/mL) | 1 | 7 | 8 | 5 |

### 2. Blood sample collection

Blood samples for CTC analysis were obtained from CRC patients before curative resection of tumor. Sampling of blood from the antecubital veins of patients with CRC was conducted before surgery. During surgery, mesenteric venous blood samples were drawn from the main drainage vein of the diseased segment of the colon, for example, the inferior mesenteric vein for cancer of the sigmoid colon or rectum or the ileocolic vein if the tumor was located on the right side of the colon. To minimize the possibility of releasing CTCs by mechanical manipulation, colonoscopy was scheduled at least 1 day before the surgery. The surgical approach sought vascular control first, that is, ligation of the feeding artery at the beginning, followed by mesenteric vein cannulation and blood drawing. The tumor was left untouched until late in the surgery. Peripheral blood (PB) and mesenteric venous blood (MVB) samples for CTC analysis were obtained from 116 patients. Blood samples for CTC analysis were also obtained from other cancer patients before drug administrations.

### 3. CTC enumeration and PD-L1 expression test on CTC

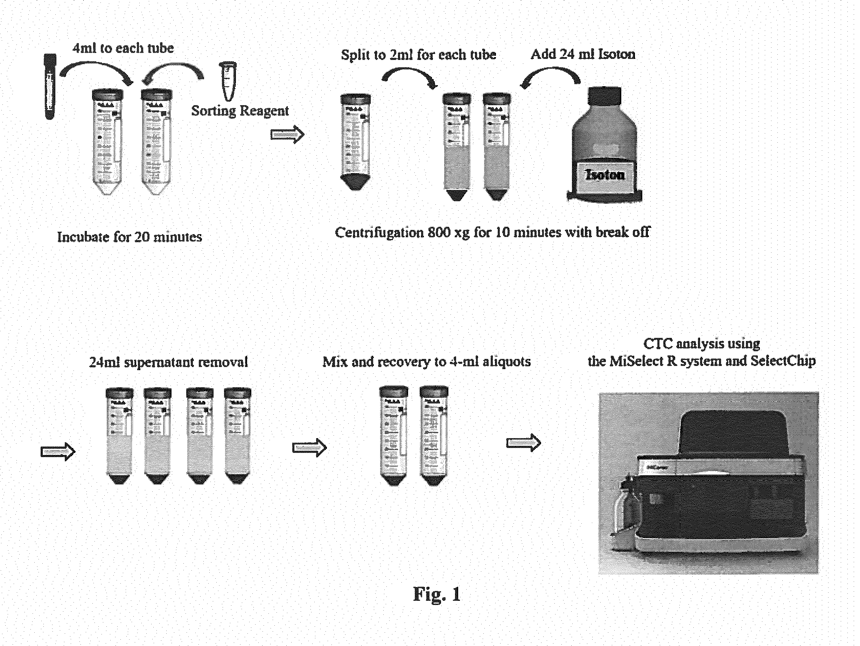

The sample preparation is shown in **Fig. 1**. Transfer two 4-ml aliquots of blood from K₂EDTA tube into two correspondingly labeled 50 ml conical centrifuge tubes. Samples incubate with Sorting Reagent (PE-conjugated anti-EpCAM antibody) of SelectKit for 20 minutes at room temperature. After staining, spilt each 4ml blood to two 2-ml aliquots into two correspondingly labeled 50 ml conical centrifuge tubes, add 24 ml ISOTON Diluent into each tube. Centrifuge the sample at 800 x g for a full 10 minutes with the brake off using a swing bucket centrifuge at room temperature. Following centrifugation, remove 24 ml supernatant of each tube and mix the samples following recovery of two 2-ml aliquots into 4ml aliquots for CTC analysis. Process on the MiSelect R System within 1 hour of sample preparation.

MiSelect R System with SelectChip Dual (MiCareo Taiwan Co., Ltd) can sort and enrich CTC. Once the aliquots containing CTCs have been collected in SelectChip, blood cells, especially RBCs, are removed from the CTCs by an on-chip filtration system. After enrichment of CTCs, Fixation and Staining Reagent of SelectKit are automated added for identification and enumeration of CTCs. Anti-panCK APC is specific targeting for the intracellular protein cytokeratin, DAPI stains for the cell nucleus and anti-CD45 FITC is specific for leukocytes. An event is classified as a tumor cell when its morphological features are consistent with that of a tumor cell and it exhibits the phenotype EpCAM⁺, CK⁺, DAPI⁺, and CD45⁻.

The CTC number will be counted and analyzed by operators and recorded directly. For further immunostaining on CTCs, anti-PD-L1 will be automated injecting into SelectChip for labeling CTC on MiSelect R System. The fluorescence images of each biomarkers on CTC will be taken and intensity of the biomarkers will be recorded for further analysis.

### 4. Statistical analyses

All data were statistically analyzed with SPSS software (v19.0) and GraphPad Prism (v5.0). The distributions of continuous variables were described as median values and ranges. The Mann-Whitney U test and the Wilcoxon-signed ranks test were performed to evaluate the differences between groups, as appropriate. All P-values were two-sided. P-values of less than 0.05 indicated statistical significance.

### Results

### 1. Prevalence of CTC in PB and MVB

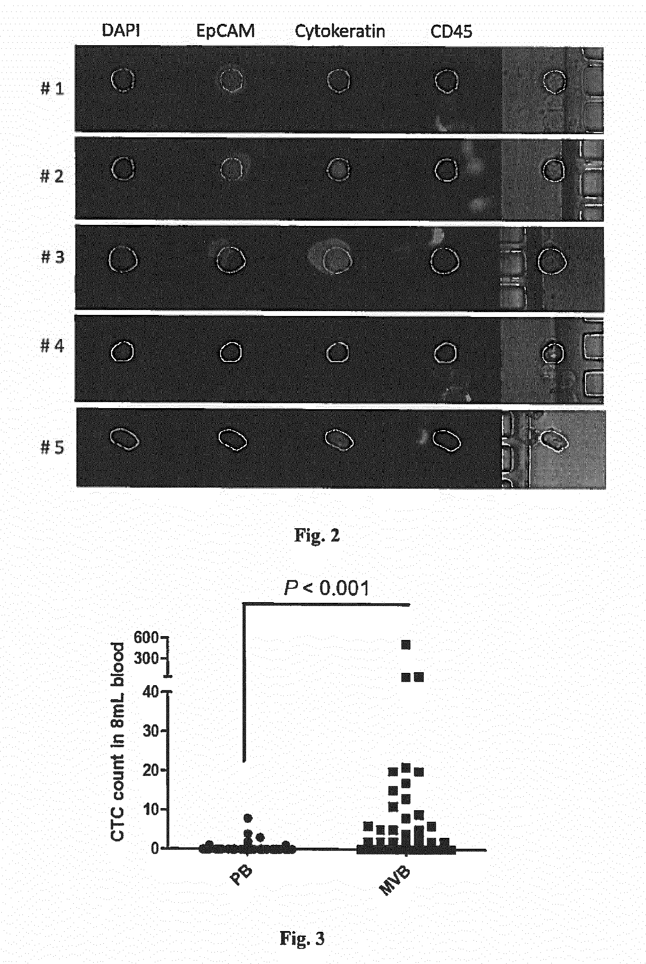

CTC was defined as a cell with intact nucleus, expressing EpCAM and cytokeratin, but absence of CD45 expression. Typical CTC images were demonstrated in **Fig. 2****.** The EpCAM expression showed heterogeneity among CTCs even within the same CRC patient. CTCs were barely found in PB (mean, 0.17 ± 0.89 per 8 ml of whole blood; range = 0-8, n = 116) but more abundant in MVB (mean, 7.1 ± 48.1 per 8 ml of whole blood; range = 0-515, n = 116) (**Fig**. **3****;** P < 0.001).

### 2. Distribution of CTC counts and detection rates

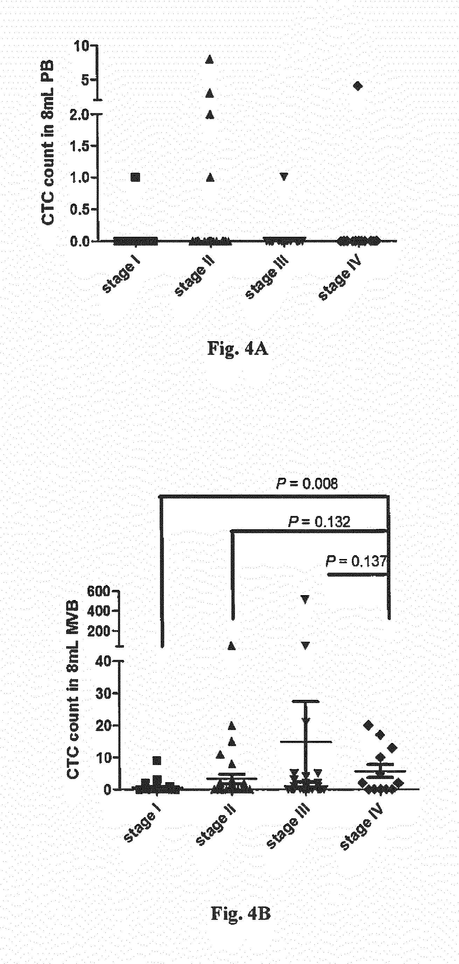

The CTC counts for CRC patients are presented in **Table 2** below and in **Fig. 4****.** CTC count ranged from 0 to 8 in non-metastatic CRC and 0 to 4 in metastatic CRC in PB; In MVB, CTC count ranged from 0 to 515 in non-metastatic CRC and 0 to 20 in metastatic CRC. The overall detection rate of CTC is 6% and 40.5% in PB and MVB, respectively. Within each subgroup, the detection rate increased with the severity of the subgroup's condition. In MVB, CTC detection rate was 20.8%, 42.1%, 45.2% and 58.3% for stage I, II, III, and IV respectively. Besides, the amount of CTC was significantly more abundant in late stages than early stages (**Fig. 4**).

**Table 2. CTC count in various stages of PB and MVB**

| | No. of cases N= 116 | Detection rate in MVB % | Range of CTC number in 8mL |
|---|---|---|---|
| Stage I | 24 | 20.8% (5/24) | 0-9 |
| Stage II | 38 | 42.1% (16/38) | 0-20 |
| Stage III | 42 | 45.2% (19/42) | 0-515 |
| Stage IV | 12 | 58.3% (7/12) | 0-20 |
| P-value = 0.0298 | | | |
| | | | |
| | No. of cases N= 116 | Detection rate in PB % | Range of CTC number in 8mL |
| Stage I | 24 | 4.2% (1/24) | 0-1 |
| Stage II | 38 | 10.5% (4/38) | 0-8 |
| Stage III | 42 | 2.4% (1/42) | 0-1 |
| Stage IV | 12 | 16.6% (2/12) | 0-4 |
| P-value = 0.7053 | | | |

### 3. Relationships between CTC number and clinicopathological characteristics

We explored the bivariate relationship between CTC numbers (present or absent) versus various clinical and pathological parameters. The results are shown in **Table 3** below. The clinical staging (TNM) positively correlated to CTC number in MVB **(Table 2)** and pre-operative serum CEA level and tumor invasion depth (pT) positively correlated to CTC levels in MVB. No association was noted between CTC numbers and presence of liver or lung metastases, primary CRC differentiation, histology, nodal status (N), lymphatic/venous invasion/perineural invasion, inflammatory change around carcinoma, invasion pattern of cancer tissue and pre-operative serum CA-19-9 level.

**Table 3. Correlation between clinicopathological parameter and the present of CTCs**

| Clinicopathological variables | CTC present % | CTC absent % | P value |
|---|---|---|---|
| T stage | 17% (6/34) | 83% (28/34) | 0.018 |
| T1/T2 | | | |
| T3/T4 | 48%(41/84) | 52% (43/84) | |
| N stage | | | 0.13 |
| N0 | 34% (22/65) | 66%(43/65) | |
| N1 | 49% (25/51) | 51% (26/51) | |
| CEA | | | 0.049 |
| ≥ 5mg/mL | 53% (23/44) | 47% (21/44) | |
| < 5mg/mL | 33% (23/71) | 67% (48/71) | |
| CA-199 | | | 0.33 |
| ≥ 37mg/mL | 50% (11/22) | 50%(11/22) | |
| < 37mg/mL | 37%(35/94) | 63% (49/94) | |

### 4. PD-L1 biomarker assessment on CTCs in CRC patients

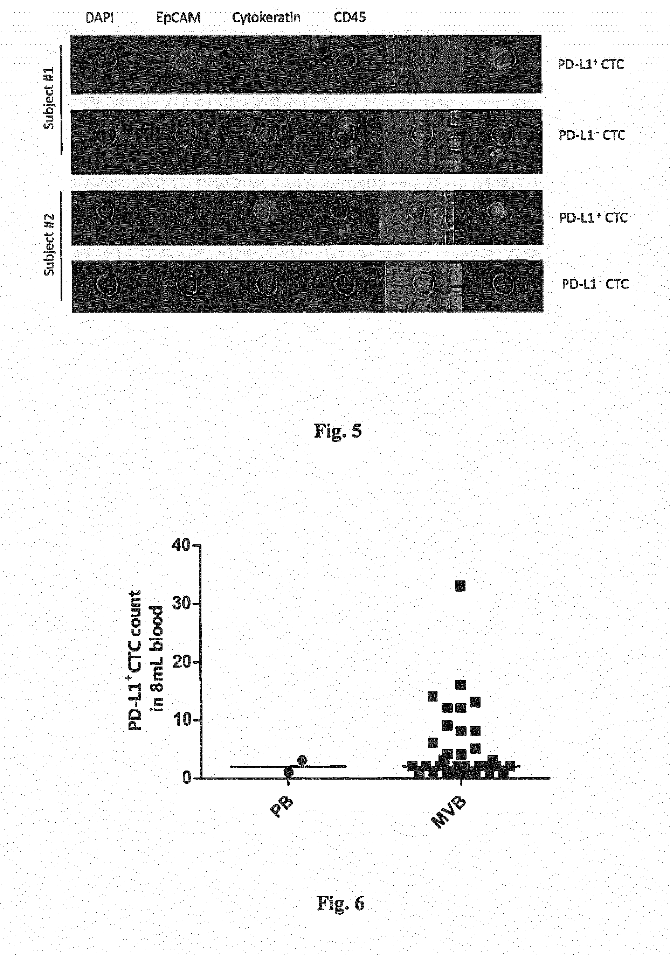

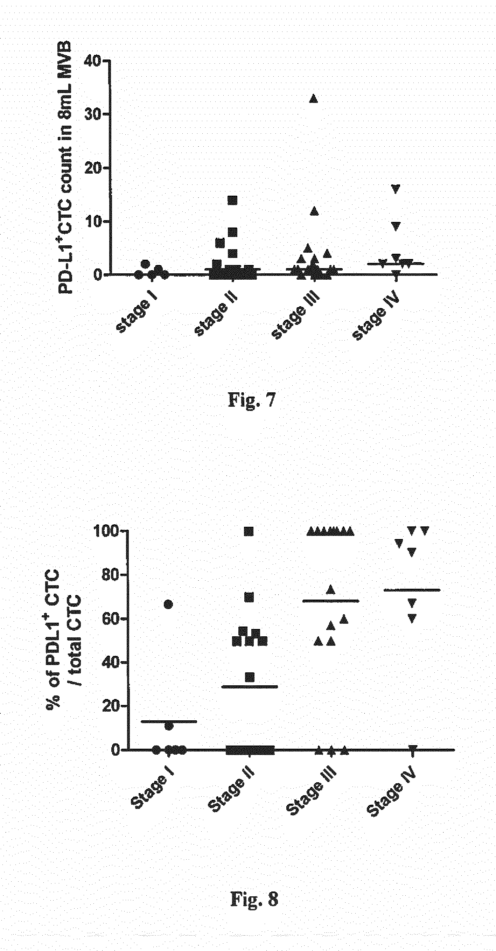

CTCs isolated from PB and MVB were examined for PD-L1 protein expression. The PD-L1 biomarker expression showed heterogeneity among isolated CTCs between patients and within the same blood sample (**Fig. 5**). PD-L1 status on CTC in PB was evaluated in 8 patients with detectable CTC (see **Table 4** below). PD-L1 status on CTC in MVB was evaluated in 47 patients with detectable CTC. Among these 47 patients, 31 (65.9%) showed a subpopulation of PD-L1⁺ CTCs (see **Fig. 6** **and Table 5** below). The number of PD-L1⁺ CTCs varied from 1 to 33 (median = 3) and the fraction of PD-L1⁺ CTCs ranged from 16 to 100% of the whole number of detectable CTCs. The PD-L1⁺ CTC number gradually increased with stages (**Fig. 7**) and the frequency of PD-L1⁺ CTCs among CTCs also increased with stages (**Fig. 8**).

**Table 4. Number of PD-L1⁺ CTC in PB of various stages**

| Patients number | Stage | CTC number | PD-L1⁺CTC number |
|---|---|---|---|
| 0031 | I | 1 | 0 |
| 0009 | II | 1 | 0 |
| 0078 | II | 1 | 0 |
| 0090 | II | 2 | 1 |
| 0113 | II | 3 | 0 |
| 0057 | III | 1 | 0 |
| 0004 | IV | 1 | 0 |
| 0075 | IV | 4 | 3 |

**Table 5. Number of PD-L1⁺ CTC in MVB of various stages**

| Patients number | Stage | CTC number | PD-L1⁺CTC number |
|---|---|---|---|
| 0011 | I | 1 | 0 |
| 0021 | I | 3 | 2 |
| 0031 | I | 1 | 0 |
| 0123 | I | 2 | 0 |
| 0129 | I | 9 | 1 |
| 0001 | II | 5 | 0 |
| 0009 | II | 2 | 2 |
| 0016 | II | 1 | 0 |
| 0037 | II | 2 | 1 |
| 0046 | II | 2 | 1 |
| 0051 | II | 2 | 0 |
| 0055 | II | 8 | 4 |
| 0060 | II | 1 | 0 |
| 0089 | II | 1 | 0 |
| 0091 | II | 1 | 0 |
| 0094 | II | 11 | 6 |
| 0096 | II | 2 | 0 |
| 0097 | II | 1 | 0 |
| 0103 | II | 15 | 8 |
| 0118 | II | 3 | 1 |
| 0139 | II | 20 | 14 |
| 0006 | III | 1 | 0 |
| 0012 | III | 4 | 4 |
| 0025 | III | 1 | 0 |
| 0030 | III | 1 | 1 |
| 0057 | III | 1 | 1 |
| 0069 | III | 5 | 5 |
| 0076 | III | 1 | 0 |
| 0077 | III | 3 | 3 |
| 0082 | III | 45 | 33 |
| 0084 | III | 21 | 12 |
| 0085 | III | 5 | 3 |
| 0086 | III | 2 | 1 |
| 0098 | III | 2 | 1 |
| 0100 | III | 1 | 1 |
| 0102 | III | 1 | 1 |
| 0104 | III | 2 | 1 |
| 0106 | III | 1 | 1 |
| 0134 | III | 1 | 0 |
| 0141 | III | 6 | 2 |
| 0004 | IV | 5 | 0 |
| 0017 | IV | 17 | 16 |
| 0028 | IV | 13 | 2 |
| 0034 | IV | 5 | 3 |
| 0038 | IV | 2 | 2 |
| 0068 | IV | 2 | 2 |
| 0075 | IV | 10 | 9 |

### 5. Relationships between PD-L1⁺ CTC number and clinicopathological characteristics in CRC patients

We explored the bivariate relationship between PD-L1⁺CTC numbers (present or absent) versus various clinical and pathological parameters. The results are shown in **Table 6** and **Table 7** below. The clinical staging (TNM) positively correlated to PD-L1⁺CTC number in MVB **(Table 6)** and pre-operative serum CEA level, tumor invasion depth (pT), nodal status (N), positively correlated to PD-L1⁺CTC levels in MVB. Besides, the primary CRC lymphatic and venous invasion were correlated to PD-L1⁺CTC levels in MVB **(Table 7**). No association was noted between PD-L1⁺CTC numbers and primary CRC differentiation, perineural invasion, inflammatory change around carcinoma, invasion pattern of cancer tissue and pre-operative serum CA-19-9 level.

**Table 6. Correlation between PD-L1(+) CTC presence and clinical stage**

| | Detection rate of PD-L1(+) CTC | PD-L1(+) rate in CTC detected patients |
|---|---|---|
| | N = 116 | N = 47 |
| Stage I | 8.3% (2/24) | 40.0% (2/5) |
| Stage II | 21.1% (8/38) | 50.0% (8/16) |
| Stage III | 35.7% (15/42) | 78.9% (15/19) |
| Stage IV | 50.0% (6/12) | 85.7% (6/7) |
| | P-value = 0.0017 | P-value = 0.0178 |

**Table 7. Correlation between clinicopathological parameter and the present of PD-L1⁺ CTCs**

| Clinicopathological variables | PD-L1(+) CTC present (%) | PD-L1(+) CTC absent (%) | P value |
|---|---|---|---|
| T stage | | | 0.016 |
| T1/T2 | 6% (2/33) | 94% (31/33) | |
| T3/T4 | 48% (23/83) | 52% (60/83) | |
| N stage | | | 0.013 |
| N0 | 12% (8/65) | 88% (57/65) | |
| N1 | 33% (17/51) | 67% (34/51) | |
| CEA | | | 0.002 |
| ≥ 5mg/mL | 36% (17/47) | 64% | |
| < 5mg/mL | 10% (7/67) | (30/47)90%(7/67) | |
| CA-199 | | | 0.13 |
| ≥ 37mg/mL | 33% (7/21) | 67% (14/21) | |
| < 37mg/mL | 17% (16/92) | 83% (76/92) | |
| Blood Vascular Invasion | | | 0.014 |
| (+) | 42% (11/26) | 58% (15/26) | |
| (- ) | 17% (14/84) | 83% (70/84) | |
| Lymphatic Vascular Invasion | | | 0.0042 |
| (+) | 36% (11/30) | 64% (19/30) | |
| (- ) | 18% (14/80) | 82% (66/80) | |

### 6. PD-L1 biomarker assessment on CTCs in other cancer patients

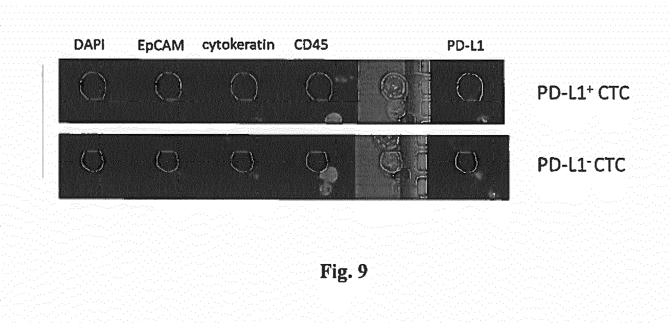

The overall detection rate of CTC is 31%, 55% and 40% in patients with head and neck squamous-cell carcinoma (HNSCC), hepatocellular carcinoma (HCC) and uterine cancer (UC), respectively (see **Table 8** below). CTCs isolated from HNSCC, HCC and UC patients were examined for PD-L1 protein expression. The PD-L1 biomarker expression showed heterogeneity among isolated CTCs between patients and within the same blood sample (**Fig. 9**). PD-L1 status on CTC was evaluated in patients with detectable CTCs. Among these patients, 100% of HNSCC, 50% of HCC and 100% of UC showed a subpopulation of PD-L1⁺ CTCs (**Table 8**)**.** The presence of PD-L1(+) CTC significantly correlates with disease progression in HNSCC patients (see **Table 9** below).

**Table 8. CTC and PD-L1(+) CTC detection rate in advanced cancers**

| Cancer type | CTC detection rate % | PD-L1(+) rate in CTC detected patients % | PD-L1 positive rate in CTC (Range) |
|---|---|---|---|
| HNSCC | 31% (8/26) | 100% (8/8) | 12.5-100% |
| N=26 | | | |
| HCC | 55% (4/9) | 50% (2/4) | 50-100 % |
| N=9 | | | |
| UC | 40% (2/5) | 100% (2/2) | 100% |
| N=5 | | | |
| RCC | 0/2 | 0% | N.A. |
| N=2 | | | |

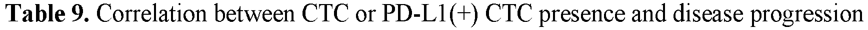

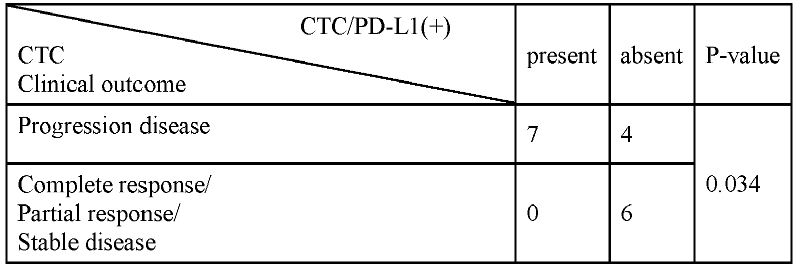

References:
1. Pantel, K., C. Alix-Panabieres, and S. Riethdorf, Nat Rev Clin Oncol, 2009. 6(6): p. 339-51.
2. Cohen, S.J., et al., J Clin Oncol, 2008. 26(19): p. 3213-21.
3. Thorsteinsson, M., G. Soletormos, and P. Jess, Anticancer Res, 2011. 31(2): p. 613-7.
4. Sastre, J., et al., Ann Oncol, 2008. 19(5): p. 935-8.
5. Nicolazzo, C. et al., Sci Rep. 2016 Aug 24;6:31726.

## Claims

1. A method for identifying a subject at risk of developing a recurrent or metastatic cancer, comprising detecting PD-L1⁺ circulating tumor cells in a mesenteric venous blood sample of the subject, wherein the presence of one or more PD-L1⁺ circulating tumor cells indicates that the subject is at risk of developing a recurrent or metastatic cancer, and wherein the subject has a gastrointestinal cancer or head and neck squamous-cell carcinoma and the blood sample is derived from the subject before a therapy for, or during a surgery of curative resection of the gastrointestinal cancer or head and neck squamous-cell carcinoma.

2. The method of claim 1, wherein the cancer is a gastrointestinal cancer.

3. The method of claim 2, wherein the gastrointestinal cancer is a colorectal cancer.

4. The method of claim 1, wherein the mesenteric venous blood sample is derived from the subject during a surgery of curative resection of the gastrointestinal cancer.

5. A method for predicting prognosis of a patient having gastrointestinal cancer or head and neck squamous-cell carcinoma, comprising detecting PD-L1⁺ circulating tumor cells in a mesenteric venous blood sample of the patient, wherein the presence of one or more PD-L1⁺ circulating tumor cells indicates poor prognosis of the patient, and wherein the blood sample is derived from the patient before a therapy for, or during a surgery of curative resection of the gastrointestinal cancer or head and neck squamous-cell carcinoma.

6. The method of claim 5, wherein the patient has a gastrointestinal cancer.

7. The method of claim 6, wherein the gastrointestinal cancer is a colorectal cancer.

8. The method of claim 5, wherein the mesenteric venous blood sample is derived from the subject during a surgery of curative resection of the gastrointestinal cancer.

## Patentansprüche

1. Verfahren zum Identifizieren einer Person mit dem Risiko, einen rezidivierenden oder metastasierenden Krebs zu entwickeln, welches das Nachweisen von PD-L1⁺ zirkulierenden Tumorzellen in einer mesenterialen Venenblutprobe der Person umfasst, wobei das Vorhandensein von einer oder mehreren PD-L1⁺ zirkulierenden Tumorzellen anzeigt, dass die Person ein Risiko hat, einen rezidivierenden oder metastasierenden Krebs zu entwickeln, und worin die Person einen gastrointestinalen Krebs oder ein Plattenepithelkarzinom des Hals-Kopf-Bereiches hat und die Blutprobe von der Person vor einer Therapie für oder während einer Operation zur kurativen Resektion des gastrointestinalen Krebses oder des Plattenepithelkarzinoms des Hals-Kopf-Bereiches gewonnen wurde.

2. Verfahren nach Anspruch 1, worin der Krebs ein gastrointestinaler Krebs ist.

3. Verfahren nach Anspruch 2, worin der gastrointestinale Krebs ein kolorektaler Krebs ist.

4. Verfahren nach Anspruch 1, worin die mesenteriale Venenblutprobe von der Person während einer Operation zur kurativen Resektion des gastrointestinalen Krebses gewonnen wurde.

5. Verfahren zur Vorhersage der Prognose eines Patienten mit Magen-Darm-Krebs oder Plattenepithelkarzinom des Hals-Kopf-Bereiches, welches den Nachweis von PD-L1⁺ zirkulierenden Tumorzellen in einer mesenterialen Venenblutprobe des Patienten umfasst, worin das Vorhandensein von einer oder mehreren PD-L1⁺ zirkulierenden Tumorzellen eine schlechte Prognose für den Patienten anzeigt, und worin die Blutprobe von dem Patienten vor einer Therapie für oder während einer Operation zur kurativen Resektion des gastrointestinalen Krebses oder Plattenepithelkarzinoms des Hals-Kopf-Bereiches gewonnen wurde.

6. Verfahren nach Anspruch 5, worin der Patient einen gastrointestinalen Krebs hat.

7. Verfahren nach Anspruch 6, worin der gastrointestinale Krebs ein kolorektaler Krebs ist.

8. Verfahren nach Anspruch 5, worin die mesenteriale Venenblutprobe von der Person während einer Operation zur kurativen Resektion des gastrointestinalen Krebses gewonnen wurde.

## Revendications

1. Méthode d'identification d'un sujet à risque de développer un cancer récurrent ou métastatique, comprenant la détection de cellules tumorales circulantes PD-L1⁺ dans un échantillon de sang veineux mésentérique du sujet, dans lequel la présence d'une ou plusieurs cellules tumorales circulantes PD-L1⁺ indique que le sujet est à risque de développer un cancer récurrent ou métastatique, et dans lequel le sujet est atteint d'un cancer gastro-intestinal ou d'un carcinome épidermoïde de la tête et du cou et l'échantillon de sang est prélevé sur le sujet avant une thérapie ou pendant une intervention chirurgicale de résection curative du cancer gastro-intestinal ou du carcinome épidermoïde de la tête et du cou.

2. Méthode de la revendication 1, dans laquelle le cancer est un cancer gastro-intestinal.

3. Méthode de la revendication 2, dans laquelle le cancer gastro-intestinal est un cancer colorectal.

4. Méthode de la revendication 1, dans laquelle l'échantillon de sang veineux mésentérique est prélevé sur le sujet au cours d'une intervention chirurgicale de résection curative du cancer gastro-intestinal.

5. Méthode de prédiction du pronostic d'un patient atteint d'un cancer gastro-intestinal ou d'un carcinome épidermoïde de la tête et du cou, comprenant la détection de cellules tumorales circulantes PD-L1⁺ dans un échantillon de sang veineux mésentérique du patient, dans lequel la présence d'une ou plusieurs cellules tumorales circulantes PD-L1⁺ indique un mauvais pronostic pour le patient, et dans lequel l'échantillon de sang est prélevé sur le patient avant une thérapie ou pendant une intervention chirurgicale de résection curative du cancer gastro-intestinal ou du carcinome épidermoïde de la tête et du cou.

6. Méthode de la revendication 5, dans laquelle le patient est atteint d'un cancer gastro-intestinal.

7. Méthode de la revendication 6, dans laquelle le cancer gastro-intestinal est un cancer colorectal.

8. Méthode de la revendication 5, dans laquelle l'échantillon de sang veineux mésentérique est prélevé sur le sujet au cours d'une intervention chirurgicale de résection curative du cancer gastro-intestinal.
